# EUROPEAN PATENT APPLICATION

(11) **EP 4 477 744 A1**
(43) Date of publication of application: **18.12.2024**
(21) Application number: 23749881.1
(22) Date of filing: 06.02.2023
(51) Int. Cl.: C12N 5/071, C07K 14/33

(54) **METHOD FOR INDUCING DIFFERENTIATION OF PLURIPOTENT STEM CELLS INTO RETINAL PIGMENT EPITHELIAL CELLS**

(30) Priority: 07.02.2022 JP 2022017547
(71) Applicant: OSAKA UNIVERSITY, Suita-shi Osaka 565-0871 (JP)
(72) Inventor: KINOOKA, Masahiro, Suita-shi, Osaka 565-0871 (JP); KIM, Meehae, Suita-shi, Osaka 565-0871 (JP); FUJINAGA, Yukako, Suita-shi, Osaka 565-0871 (JP)
(74) Representative: Hoffmann Eitle
(86) International application number: PCT/JP2023/003688
(87) International publication number: WO 2023/149565

(57) **Abstract**

A method for producing a retinal pigment epithelial (RPE) cell, including promoting differentiation induction from a pluripotent stem cell into an RPE cell by culturing the pluripotent stem cell in a medium containing an E-cadherin inhibitor is provided by the present invention.

## Description

### [Technical Field]

The present invention relates to a method for uniformly and homogeneously, and efficiently inducing differentiation of pluripotent stem cells into retinal pigment epithelial cells by using hemagglutinin, namely, a method for efficiently producing uniform and homogeneous retinal pigment epithelial cells from pluripotent stem cells. The present invention also relates to a promoting agent that contains hemagglutinin and induces differentiation of pluripotent stem cells into retinal pigment epithelial cells, and further, a reagent or kit for inducing differentiation of pluripotent stem cells into retinal pigment epithelial cells that contains a substance that induces differentiation of pluripotent stem cells into retinal pigment epithelial cells (differentiation inducer), and the like.

### [Background Art]

In the stem cell industry, including regenerative medicine, it is necessary to produce differentiated cells of interest in vitro in large amounts and in a uniform and homogeneous manner. As a cell source in this case, pluripotent stem cells may be used. In this cell production (differentiation induction) process, it is necessary to induce differentiation of pluripotent stem cells into target cells, mature the cells, and maintain the differentiation induction state as uniformly as possible.

However, in the process of inducing differentiation from pluripotent stem cells into the desired cells in vitro, undesired cells may be generated. In addition, the speed of differentiation induction may vary between individual cells, and uniformly differentiated cells may not be obtained. Furthermore, even if differentiation induction is performed under the same conditions, the quality of the cells obtained may vary depending on the technique.

When induction of differentiation from pluripotent stem cells into the desired cells in vitro is attempted, it is sometimes very difficult to obtain a population of uniformly and homogeneously differentiated cells. While many methods for differentiation induction and many methods for increasing the induction efficiency have been reported, there is still no useful universal method for obtaining a uniform cell population, which forms a major obstacle to the production of uniform and high-quality cellular medicines.

For example, for diseases such as age-related macular degeneration, the establishment of a treatment method using retinal pigment epithelial cells (hereinafter sometimes to be abbreviated as "RPE cells") derived from pluripotent stem cells is underway. In the production of cellular medicines using RPE cells, a major problem is how to remove fibroblast-like cells that may be included as undesired cells. The fibroblast-like cell groups are cells that can be generated in the course of normal differentiation induction, and how to produce the desired RPE cells without generating these cells is very important to ensure the quality of the final product.

As methods for inducing differentiation of pluripotent stem cells into RPE cells, methods using Nodal signal inhibitors and Wnt signal inhibitors (Patent Literature 1, Patent Literature 2), the so-called SFEB method (Patent Literature 3), a method using FGF receptor inhibitors and MEK inhibitors (Patent Literature 4), and methods for culturing pluripotent stem cells under feeder-free conditions and using the E8 fragment of laminin 511 (Patent Literature 5), laminin 111 and Matrigel (Non-Patent Literature 1), as substrates are known. However, none of these methods is a universal method for obtaining a uniform population of desired RPE cells.

In the differentiation induction process of pluripotent stem cells, attempts have been made to increase the production amount of a certain differentiated cell or improve the efficiency of differentiation induction by using an E-cadherin inhibitor (Patent Literatures 6 and 7).

Patent Literature 6 discloses a method of acting an E-cadherin inhibitor on human embryonic stem (ES) cells and improving the efficiency of differentiation into definitive endoderm cells by using TGFβ, thereby increasing the production amount of the cells. In addition, Patent Literature 7 discloses that, in a method of inducing differentiation of ES or induced pluripotent stem (iPS) cells, E-cadherin inhibitors delay differentiation into most cell lineages but do not delay differentiation into neural progenitor cells, and as a result, the E-cadherin inhibitors contribute to the induction of differentiation into neural progenitor cells.

On the other hand, the present inventors have reported that, in the maintenance culture process of pluripotent stem cells, hemagglutinin or a variant thereof, which is an E-cadherin inhibitor, was used to maintain an undifferentiated state, thereby enabling the mass culture of the pluripotent stem cells (Patent Literatures 8 to 10).

Patent Literatures 8 and 9 disclose methods for removing cells that have deviated from the undifferentiated state and have occurred or may occur during the culture of pluripotent stem cells, by using hemagglutinin or a variant thereof. Patent Literature 9 discloses a method for dividing cell aggregates in suspension culture of iPS cells into small clumps by using hemagglutinin. Furthermore, Patent Literature 10 discloses a method for enabling mass culture of pluripotent stem cells by using hemagglutinin or a variant thereof.

However, there are no reports that E-cadherin inhibitors contribute to differentiation induction of pluripotent stem cells, other than Patent Literatures 6 and 7 showing differentiation induction into definitive endoderm cells or neural precursor cells. In addition, the inventions described in Patent Literatures 8 to 10 are techniques relating to the maintenance and expansion culture of pluripotent stem cells, and are not techniques relating to differentiation induction.

### [Citation List]

### [Patent Literature]

[Patent Literature 1]
   WO2008/087917
[Patent Literature 2]
   US2013/0224156
[Patent Literature 3]
   WO2005/123902
[Patent Literature 4]
   WO2015/053375
[Patent Literature 5]
   WO2017/043605
[Patent Literature 6]
   WO2009/041984
[Patent Literature 7]
   WO2014/072720
[Patent Literature 8]
   WO2014/104207
[Patent Literature 9]
   WO2015/199243
[Patent Literature 10]
   WO2018/117110

### [Non Patent Literature]

[Non Patent Literature 1]
J. Tissue Eng Regen Med 2013; 7: 642-653

### [Summary of Invention]

### [Technical Problem]

As described above, various methods have been investigated for inducing differentiation of pluripotent stem cells into RPE cells. When considering use as a cellular medicine, however, none of them is satisfactory, and establishment of a method capable of producing a more uniform RPE cell population has been desired.

Therefore, it is an object of the present invention to provide a novel differentiation induction method that makes it possible to provide RPE cells as a uniform and homogeneous cell population from pluripotent stem cells while suppressing the generation of undesired cells. It is another object of the present invention to provide a substance that can be used in the differentiation induction method and that promotes differentiation induction from pluripotent stem cells into RPE cells.

### [Solution to Problem]

When conventional differentiation induction methods are used in inducing differentiation of pluripotent stem cells into RPE cells in vitro, variations in cell growth and differentiation state occur, resulting in the occurrence of undesired cells. The present inventors considered that this might be because the degree of intercellular adhesion varies depending on the position within the aggregate during the process of proliferation and formation of aggregates (colonies) of the pluripotent stem cells. More specifically, the present inventors made a hypothesis that the cells are not so crowded at the periphery of the colony or aggregate, and the intercellular adhesion is not strong, but inside the colony or aggregate, the cells are crowded and strongly adhere to each other, and as a result, the differentiation induction environment for the pluripotent stem cells that constitute the colony or aggregate is different, resulting in the occurrence of cells that have deviated from the desired differentiation into RPE cells.

The present inventors thus attempted to make uniform the intercellular adhesive force in the aggregates by adding hemagglutinin capable of inhibiting E-cadherin, which is a protein that controls intercellular adhesion, to a differentiation induction system from pluripotent stem cells into RPE cells. As a result, the addition of an appropriate amount of hemagglutinin improved the efficiency of RPE cell differentiation induction and suppressed the generation of undesired cells.

Based on these findings, the present inventors further conducted intensive studies and completed the present invention.

That is, the present invention provides the following.

### (Item 1)

A method for producing a retinal pigment epithelial (RPE) cell, comprising promoting differentiation induction from a pluripotent stem cell into an RPE cell by culturing the pluripotent stem cell in a medium comprising anE-cadherin inhibitor.

### (Item 2)

The method of Item 1, wherein the pluripotent stem cell is an embryonic stem (ES) cell or an induced pluripotent stem (iPS) cell.

### (Item 3)

The method of Item 1 or 2, wherein the medium comprises a differentiation inducer.

### (Item 4)

The method of Item 3, wherein the differentiation inducer is a Nodal signal inhibitor and a Wnt signal inhibitor.

### (Item 5)

The method of Item 1 or 2, comprising the following steps:
(1) a step of forming an aggregate of pluripotent stem cells, and
(2) a step of culturing the aggregates obtained in step (1) in an RPE cell induction medium comprising an E-cadherin inhibitor.

### (Item 6)

The method of Item 5, wherein the RPE cell induction medium comprises a Nodal signal inhibitor and a Wnt signal inhibitor.

### (Item 7)

The method of any one of Items 1 to 6, wherein the E-cadherin inhibitor is hemagglutinin.

### (Item 8)

A differentiation induction promoting agent from a pluripotent stem cell into an RPE cell, comprising an E-cadherin inhibitor.

### (Item 9)

The promoting agent of Item 8, wherein the pluripotent stem cell is an ES cell or an iPS cell.

### (Item 10)

The promoting agent of Item 8 or 9, wherein the E-cadherin inhibitor is hemagglutinin.

### (Item 11)

A reagent or kit for inducing differentiation of a pluripotent stem cell into an RPE cell, comprising an E-cadherin inhibitor and a differentiation inducer.

### (Item 12)

The reagent or kit of Item 11, wherein the differentiation inducer is a Nodal signal inhibitor and a Wnt signal inhibitor.

### (Item 13)

The reagent or kit of Item 11 or 12, wherein the pluripotent stem cell is an ES cell or an iPS cell.

### (Item 14)

The reagent or kit of any one of Items 11 to 13, wherein the E-cadherin inhibitor is hemagglutinin.

### [Advantageous Effects of Invention]

According to the present invention, uniform and homogeneous RPE cell population can be produced from pluripotent stem cells efficiently and reproducibly.

### [Description of Embodiments]

The present invention is based, at least in part, on the previous study results of the present inventors, showing that cells that have deviated from an undifferentiated state occur within a colony in the maintenance and expansion culture of pluripotent stem cells because the strength of intercellular adhesion force caused by E-cadherin varies within the colony, which in turn causes ununiform sensitivity of each pluripotent stem cell to the medium environment that supports the maintenance of the undifferentiated state. In other words, based on the findings obtained by the maintenance and expansion technology of pluripotent stem cells, the present inventors took note of the inhibition of intercellular adhesion by E-cadherin inhibitors, and assumed that this inhibition also contributes to the "uniformization and homogenization" of the intracellular environment during the differentiation induction of pluripotent stem cells, and, as a result, affords qualitative and quantitative improvements also in the RPE cells obtained in the differentiation induction system from pluripotent stem cells into RPE cells. This hypothesis may explain the apparently contradictory phenomenon that E-cadherin inhibitors not only maintain the undifferentiated state of pluripotent stem cells, but also contribute to differentiation induction into certain differentiated cells (embryonic endoderm cells and neural precursor cells).

The contents of the present invention are described in detail below.

### 1. Differentiation induction method of the present invention

The present invention provides a method for producing RPE cells from pluripotent stem cells, in other words, a method for inducing differentiation of pluripotent stem cells into RPE cells. Specifically, the present invention provides a method for inducing differentiation of pluripotent stem cells into RPE cells, including a step of culturing pluripotent stem cells in a medium containing an E-cadherin inhibitor. In addition, the present invention provides a method for inducing differentiation of pluripotent stem cells into RPE cells, including (1) a step of forming an aggregate of pluripotent stem cells, and (2) a step of culturing the aggregates obtained in step (1) in an RPE cell induction medium containing an E-cadherin inhibitor. In the following, they are at times collectively referred to as "the differentiation induction method of the present invention".

### [Pluripotent stem cell]

In the present specification, the pluripotent stem cell is not particularly limited as long as it has the "self-renewal ability" to proliferate while maintaining an undifferentiated state and the "differentiation pluripotency" to differentiate into all three germ lineages. Examples thereof include induced pluripotent stem cells (iPS cells) and embryonic stem cells (ES cells). In addition, examples of the pluripotent stem cell include pluripotent stem cells derived from mammals. Pluripotent stem cells derived from mammals are not particularly limited, and examples thereof include human pluripotent stem cells. Human pluripotent stem cells are not particularly limited, and examples thereof include human iPS cells and human ES cells.

### [Retinal pigment epithelial (RPE) cell]

In the present specification, the retinal pigment epithelial (RPE) cell refers to an epithelial cell constituting the retinal pigment epithelium, and a progenitor cell thereof. Whether or not the cell is an RPE cell can be confirmed, for example, based on the expression of cell markers (RPE65, CRALBP, MERTK, BEST1, etc.), the cell morphology (intracellular melanin pigment deposition, polygonal and flat cell morphology, formation of polygonal actin bundle, etc.), and the like. The progenitor cell of RPE cell means a cell directed to be induced to differentiate into retinal cell, and whether the progenitor cell or not can be confirmed by expression of cell markers (Mitf, Pax6, Rx, Crx, etc.) and the like. Functional evaluation of RPE cell can be confirmed using, for example, secretability, phagocytic capacity and the like of cytokines (VEGF, PEDF etc.) as an index. These functional evaluation and confirmation operations can be performed by those of ordinary skill in the art by setting appropriate conditions.

### [E-cadherin inhibitor]

In the present specification, the E-cadherin inhibitor may be any substance capable of inhibiting intercellular adhesion caused by E-cadherin, and examples thereof include hemagglutinin, nucleic acids capable of inhibiting the expression of E-cadherin (e.g., antisense oligonucleotides, etc.), and antibodies against E-cadherin. Hemagglutinin is preferably used as the E-cadherin inhibitor.

### [Hemagglutinin (HA)]

In the present specification, the hemagglutinin is not particularly limited in terms of the method of acquisition, origin, and the like, as long as it can inhibit intercellular adhesion by E-cadherin. Examples thereof include hemagglutinin derived from Clostridium botulinum and hemagglutinin derived from related bacteria. Clostridium botulinum may be any of type A, type B, type C, and type D. Hemagglutinin refers to a neurotoxin complex (also referred to as hemagglutinin complex) unless otherwise specified.

Hemagglutinin may be of a wild-type, a hemagglutinin variant, or a miniaturized hemagglutinin.

### [Hemagglutinin variant]

In the present specification, hemagglutinin may be a hemagglutinin variant to the extent that it has an effect of contributing to the promotion of differentiation induction from pluripotent stem cells into RPE cells. One or more embodiments of the hemagglutinin variant include, for example, a variant in which one, two, or three amino acid sequences of subcomponents have at least one amino acid mutation. Specific examples of the variant include the variant hemagglutinin (HA) complex protein described in WO 2015/199243.

### [Miniaturized hemagglutinin]

In the present specification, moreover, hemagglutinin may be a miniaturized hemagglutinin to the extent that it has an effect of contributing to the promotion of differentiation induction from pluripotent stem cells into RPE cells. Examples of the miniaturized hemagglutinin include HA complex proteins that retain cadherin function inhibitory activity and lack all or part of the subcomponents. Specific examples of miniaturized hemagglutinin include the miniaturized hemagglutinin complex protein having an E-cadherin function inhibitory activity described in WO 2019/103111.

In the present specification, unless particularly specified, "hemagglutinin" includes "hemagglutinin variant" and "miniaturized hemagglutinin". In addition, in the present specification, unless particularly specified, "hemagglutinin variant" and "miniaturized hemagglutinin" refer to those that have the effect of contributing to the promotion of differentiation induction from pluripotent stem cells into RPE cells.

### [Differentiation induction from pluripotent stem cell into RPE cell]

As described above, the differentiation induction method of the present invention has been completed from the viewpoint that the addition of an E-cadherin inhibitor to an existing induction system of differentiation into RPE cells makes the mechanical cell culture condition of intercellular adhesion uniform and homogenized, thereby qualitatively and quantitatively improving the differentiation induction system. Therefore, as described below, the method for inducing differentiation of pluripotent stem cells into RPE cells is not particularly limited as long as it includes a step of culturing pluripotent stem cells in a medium containing an E-cadherin inhibitor. In the differentiation induction method of the present invention, in order to realize uniform and homogeneous differentiation induction, the E-cadherin inhibitor may be added to a medium (containing a differentiation inducer) for inducing differentiation of pluripotent stem cells into RPE cells, or the E-cadherin inhibitor may be added to a medium (containing no differentiation inducer) that does not induce differentiation into a specific differentiated cell but does not support the maintenance of an undifferentiated state, and the pluripotent stem cells may be cultured. In the latter case, the cells obtained by the culture step can be further cultured in a medium (containing a differentiation inducer) for inducing differentiation into RPE cells. The differentiation induction medium may or may not contain an E-cadherin inhibitor, but preferably contains an E-cadherin inhibitor.

Furthermore, from the aspect of allowing the E-cadherin inhibitor to exert its function, the pluripotent stem cells preferably show intercellular adhesion at the time when the induction is started. Therefore, in one embodiment of the present invention, a method for inducing differentiation of pluripotent stem cells into RPE cells includes
(1) a step of forming an aggregate of pluripotent stem cells, and
(2) a step of culturing the aggregates obtained in step (1) in an RPE cell induction medium containing an E-cadherin inhibitor.

The medium used for culturing pluripotent stem cells is not particularly limited as long as it contains an E-cadherin inhibitor and does not support the maintenance of the undifferentiated state of pluripotent stem cells. Examples thereof include media containing basal medium, serum and/or serum alternative, an E-cadherin inhibitor, preferably hemagglutinin (particularly hemagglutinin derived from Clostridium botulinum), and other components. It is preferable that the medium does not contain a substance that supports the maintenance of the undifferentiated state (e.g., bFGF, SCF, LIF, and the like), but this may not apply as long as the medium as a whole does not support the maintenance of the undifferentiated state of pluripotent stem cells.

As the basal medium, one type of synthetic medium used generally for culturing mammalian cells or multiple types thereof in combination can be used and, for example, commercial products such as Glasgow MEM (GMEM), IMDM (Iscove's Modified Dulbecco's medium), DMEM, NeuroBasal medium, NeuroBasal-A medium, F12-Ham, Kaighn's modified F12, and the like are available.

As the serum, a serum derived from a mammal such as bovine, human, and the like can be used. The serum alternative is a low-protein replacement used instead of a serum such as FBS and the like used for cell culture. As commercially available products, for example, knockout Serum Replacement (KSR), Chemically-defined Lipid concentrated (manufactured by Gibco), Glutamax (manufactured by Gibco) and the like, and N2 and B27, which are serum alternatives for nerve cell culture, and the like are available. In the present invention, a serum alternative is preferred, and KSR is particularly preferred, from the aspect of quality control of the target cells.

The concentration of serum or serum alternative can be appropriately set within the range of, for example, 0.5 - 30% (v/v). The concentration may be constant or stepwisely changed and, for example, the concentration may be stepwisely decreased at about 1 - 4 day (preferably 4 day) intervals. For example, serum or serum alternative can be added at three-step concentrations of 20%, 15% and 10%.

The concentration of the E-cadherin inhibitor used for differentiation induction into RPE cells is not particularly limited, and can be appropriately set by a person skilled in the art according to the medium conditions described above and the conditions for differentiation induction into RPE cells to be described below. For example, when hemagglutinin is used as the E-cadherin inhibitor, the hemagglutinin concentrations described in detail below can be adopted; however, they are mere examples and the concentration should not be limited thereto.

The lower limit of the concentration of hemagglutinin is, for example, 1 nM, 2 nM, 3 nM, 4 nM, 5 nM, 6 nM, 7 nM, 8 nM, 9 nM, or 10 nM, and the upper limit thereof is, for example, 80 nM, 70 nM, 60 nM, 50 nM, 45 nM, 40 nM, 35 nM, or 30 nM. The range of the concentration of hemagglutinin is, for example, 1 nM to 80 nM, 2 nM to 70 nM, 3 nM to 60 nM, 5 nM to 50 nM, 7 nM to 40 nM, or 10 nM to 30 nM, preferably 5 nM to 50 nM, more preferably 7 nM to 40 nM, particularly preferably 10 nM to 30 nM.

When hemagglutinin is a hemagglutinin variant, the lower limit of the concentration is, for example, 1 nM, 2 nM, 3 nM, 4 nM, 5 nM, 6 nM, 7 nM, 8 nM, 9 nM, or 10 nM, and the upper limit thereof is, for example, 80 nM, 70 nM, 60 nM, 50 nM, 45 nM, 40 nM, 35 nM, or 30 nM. The concentration range is, for example, 1 nM to 80 nM, 2 nM to 70 nM, 3 nM to 60 nM, 5 nM to 50 nM, 7 nM to 40 nM, or 10 nM to 30 nM, preferably 5 nM to 50 nM, more preferably 7 nM to 40 nM, particularly preferably 10 nM to 30 nM.

When hemagglutinin is miniaturized hemagglutinin, the lower limit of the concentration is, for example, 3 nM, 4 nM, 5 nM, 6 nM, 7 nM, 8 nM, 9 nM, or 10 nM, and the upper limit thereof is, for example, 200 nM, 150 nM, 100 nM, 80 nM, 60 nM, or 50 nM. The concentration range is, for example, 3 nM to 200 nM, 5 nM to 150 nM, 7 nM to 100 nM, or 10 nM to 80 nM, preferably 5 nM to 150 nM, more preferably 7 nM to 100 nM, particularly preferably 10 nM to 80 nM.

As other components constituting a medium, in order to suppress the cell death of human pluripotent stem cells dispersed in a culture medium, Rho kinase inhibitors such as Y-27632 and the like can be used. A Rho kinase inhibitor may be added, for example, for a part or the entire period of differentiation induction step. For example, by using a medium containing no Rho kinase inhibitor in the later stage of differentiation induction step, unnecessary cells that have not been differentiated into RPE cell can be removed by cell death.

The concentration of the Rho kinase inhibitor can be appropriately selected according to the type of Rho kinase inhibitor. Specifically, when Y-27632 is used as the Rho kinase inhibitor, the concentration is, for example, 1 µM to 30 µM, preferably 5 µM to 20 µM, more preferably 7 µM to 10 µM, particularly preferably 10 µM.

The medium may further contain a differentiation inducer. The differentiation inducer may be any of the known differentiation inducers used for inducing differentiation into RPE cells. For example, Nodal signal inhibitor, Wnt signal inhibitor, Sonic hedgehog signal inhibitor, Activin signal promoting agent, and the like can be mentioned.

The Nodal signal inhibitor is not particularly limited as long as it can suppress signal transduction mediated by Nodal, and protein, nucleic acid, low-molecular-weight compound and the like can be used. Examples of the Nodal signal inhibitor include protein, peptide or nucleic acid such as Lefty-A, soluble Nodal receptor, anti-Nodal antibody, Nodal receptor inhibitor and the like; low-molecular-weight compound such as SB-431542 and the like, and the like. Particularly, a low-molecular-weight compound such as SB-431542 and the like which is easily available and shows less difference between lots is preferred.

The Wnt signal inhibitor is not particularly limited as long as it can suppress signal transduction mediated by Wnt, and protein, nucleic acid, low-molecular-weight compound and the like can be used. Examples of the Wnt signal inhibitor include protein, peptide or nucleic acid such as Dkk1, Cerberus protein, Wnt receptor inhibitor, soluble Wnt receptor, Wnt antibody, casein kinase inhibitor, dominant negative Wnt protein and the like; and low-molecular-weight compound such as CKI-7(N-(2-aminoethyl)-5-chloro-isoquinoline-8-sulfonamide), D4476(4-{4-(2,3-dihydrobenzo[1,4]dioxin-6-yl)-5-pyridin-2-yl-1H-imidazol-2-yl}benzamide), IWR-1-endo(IWR1e), IWP-2 and the like. Particularly, a low-molecular-weight compound which is easily available and shows less difference between lots is preferable. Among others, a low-molecular-weight compound having an activity to selectively inhibit casein kinase I is preferable and, for example, CKI-7, D4476 and the like can be utilized.

Examples of the Activin signal promoting agent include protein belonging to the Activin family, Activin receptor, Activin receptor agonist and the like.

The concentration of these differentiation inducers can be appropriately selected according to the kind of the differentiation inducer. Specifically, when SB-431542 is used as a Nodal signal inhibitor, the concentration is, for example, 0.01 - 50 µM, preferably 0.1 - 10 µM, more preferably 5 µM; when CKI-7 is used as a Wnt signal inhibitor, it is added at the concentration of, for example, 0.01 - 30 µM, preferably 0.1 - 30 µM, more preferably 3 µM.

In the present invention, a combination of a Nodal signal inhibitor (e.g., SB-431542) and a Wnt signal inhibitor (e.g., CKI-7) is preferably used as a differentiation inducer.

The medium can contain other components generally used for culture of mammalian cells, in addition to those mentioned above.

From the aspect of allowing the E-cadherin inhibitor to exert its function, the pluripotent stem cells preferably show intercellular adhesion at the time when the induction is started. Therefore, in one embodiment of the present invention, a method for inducing differentiation of pluripotent stem cells into RPE cells includes
(1) a step of forming an aggregate of pluripotent stem cells, and
(2) a step of culturing the aggregates obtained in step (1) in an RPE cell induction medium containing an E-cadherin inhibitor.

Examples of the medium used in step (1) include media containing basal medium, serum and/or serum alternative, an E-cadherin inhibitor, preferably hemagglutinin (particularly hemagglutinin derived from Clostridium botulinum), and components other than the differentiation inducer. Examples of the "basal medium", "serum and/or serum alternative", "E-cadherin inhibitor", "hemagglutinin", and "other components" include those similar to the ones described above.

The RPE cell induction medium used in step (2) may be, for example, the medium used in step (1) above, to which a differentiation inducer capable of inducing differentiation into RPE cells has been added. As the "differentiation inducer", those similar to the ones described above can be mentioned.

As the culture conditions for pluripotent stem cells, a method known as a method for inducing differentiation into RPE cells may be used. For example, an adherent culture method, a suspension culture method, and the like can be mentioned. As the adhesion culture method, a method using an adherent cell culture vessel, a method in which pluripotent stem cells are cultured in a state of being adhered to feeder cells (e.g., WO2001/088100), a method using a culture substrate coated with laminin E8 fragment (e.g., WO2015/053375), and the like are known. The suspension culture method refers to a method of culturing a group of pluripotent stem cells that have gathered and formed a clump (floating aggregates) in a suspended state in a culture medium (e.g., WO2008/087917, Nature. 2011 Apr 7; 472 (7341) :51-6) .

### Adhesion culture method

The differentiation induction method of the present invention can be performed by adhesion culture in the existing differentiation induction method into RPE cells. Adhesion culture is started by adding an E-cadherin inhibitor in the presence of intercellular adhesion between pluripotent stem cells on the plate. The culture vessel is not particularly limited as long as it can be used for cell culture and, for example, dish (also referred to as culture dish), petri dish and plate (microtiter plate, microplate, deep well plate etc. of 6 well, 24 well, 48 well, 96 well, 384 well, 9600 well and the like), flask, chamber slide, tube, cell factory, roller bottle, spinner flask, hollow fiber, microcarrier, bead and the like can be mentioned.

The culture substrate used in the adhesion culture method is not particularly limited as long as it is already known to be used for differentiation induction from pluripotent stem cells into RPE cells, and extracellular matrix proteins known per se such as fibronectin, vitronectin, and laminin can be mentioned. These proteins may be fragmented functional peptides or full-length proteins themselves. Among these, preferred examples include culture substrates coated with laminin E8 fragments (laminin fragments) and the like. Specific examples of the substrate include the culture substrate coated with laminin E8 fragments as described in WO2015/053375, the culture substrate coated with E8 fragments of human laminin α5β1γ1 or E8 fragments of human laminin α3β3γ2 as described in WO2011/043405, and the like.

The concentration of pluripotent stem cells to be used in the culture is not particularly limited as long as pluripotent stem cells can be uniformly seeded, and adhesion culture is possible. It is desirably a concentration of the level permitting the intercellular adhesion to become uniform by the administration of an E-cadherin inhibitor (preferably hemagglutinin) when the cells are uniformly seeded and adhesion cultured. For example, when a 9 cm dish is used, it is 1×10⁵ to 1×10⁸ cells, preferably 2×10⁵ to 5×10⁷ cells, more preferably 5×10⁵ to 9×10⁶ cells, per 1 dish.

Other culture conditions such as culture temperature and CO₂ concentration can be set as appropriate. Culture temperature is not particularly limited, and is, for example, about 30 to 40°C, preferably about 37°C. The CO₂ concentration is, for example, about 1 to 10%, preferably about 5%.

The culture period is not particularly limited as long as differentiation of pluripotent stem cells into RPE cells can be induced. It is desirably a culture period that can maintain the effect of making intercellular adhesion uniform by an E-cadherin inhibitor (preferably hemagglutinin). For example, it may be about 1 day or more, preferably about 1 to 50 days, more preferably about 3 to 40 days. This culture period can be appropriately changed according to the number of pluripotent stem cells used in the culture, culture equipment, culture substrate, culture method, and the like.

### Suspension culture method

The differentiation induction method of the present invention can be performed by suspension culture in the existing differentiation induction method into RPE cells. By forming aggregates of pluripotent stem cells, intercellular adhesion between individual pluripotent stem cells is formed, and an E-cadherin inhibitor is added to render the strength of the intercellular adhesion uniform and homogeneous inside and outside the aggregates, and then suspension culture is started.

The cell culture vessel to be used in the suspension culture method is not particularly limited as long as it enables floating culture of the cells. Examples of such cell culture vessel include flask, tissue culture flask, dish, petri dish, tissue culture dish, multidish, microplate, microwell plate, micropore, multiplate, multiwell plate, chamber slide, schale, tube, tray, culture bag, roller bottle and so on. A preferable vessel is a non- cell-adhesive culture vessel.

As the non-cell-adhesive culture vessel, a culture vessel having a surface not artificially treated to improve adhesiveness to the cell (e.g., coating treatment with extracellular matrix and the like) and the like are preferably used. A non-cell-adhesive culture vessel having a surface artificially treated to reduce adhesiveness to the cell (e.g., ultra-hydrophilic treatment) and the like may also be used.

The number of aggregates used for culture can be selected according to an existing method for inducing differentiation into RPE cells. For example, when a 96-well microwell plate is used, it is one to several per well. The same applies to the number of cells per aggregate, and is, for example, about 1×10³ to about 1×10⁵ cells.

Other culture conditions such as culture temperature and CO₂ concentration can be set as appropriate. Culture temperature is not particularly limited, and is, for example, about 30 to 40°C, preferably about 37°C. The CO₂ concentration is, for example, about 1 to 10%, preferably about 5%.

The culture period is not particularly limited as long as differentiation of pluripotent stem cells into RPE cells can be induced. For example, it may be not less than about 3 days, preferably about 3 to 40 days, more preferably about 5 to 25 days.

As described above, in one embodiment of the present invention, a method for inducing differentiation of pluripotent stem cells into RPE cells includes
(1) a step of forming an aggregate of pluripotent stem cells, and
(2) a step of culturing the aggregates obtained in step (1) in an RPE cell induction medium containing an E-cadherin inhibitor derived from Clostridium botulinum.

In step (1), the "aggregate" refers to a clump formed by assembly of cells dispersed in a medium, in which the cells are adhered to each other. Colony, clump, cell clump, embryoid body, sphere, and spheroid are also encompassed in the aggregate. Aggregates of pluripotent stem cells are formed, for example, by culturing pluripotent stem cells using a suspension culture method. Examples of the medium and suspension culture method are the same as those described above. Generally, the cells are relatively dense near the center of the aggregate, and the strength of the mechanical intercellular adhesion is high. On the other hand, the cells are relatively sparse at the periphery of the aggregate, and the strength of the mechanical intercellular adhesion is low. By adding an E-cadherin inhibitor, the strength of intercellular adhesion near the center of the aggregate is alleviated, and the strength of individual intercellular adhesion in the aggregate as a whole is averaged, whereby the mechanical cell culture environment in the differentiation induction of pluripotent stem cells becomes uniform and homogenous.

As the medium and culture method used in step (2), those similar to the ones described above can be mentioned.

### 2. The differentiation induction promoting agent of the present invention

The present invention also provides a promoting agent for inducing differentiation of pluripotent stem cells into RPE cells, including an E-cadherin inhibitor, preferably hemagglutinin. In the following, it is at times referred to as "the differentiation induction promoting agent of the present invention". The E-cadherin inhibitor, hemagglutinin, and the like used in the differentiation induction promoting agent of the present invention are appropriately as described in "1. The differentiation induction method of the present invention".

### 3. Reagent/kit for differentiation induction of the present invention

The present invention also provides a reagent or kit for inducing differentiation of pluripotent stem cells into RPE cells, including an E-cadherin inhibitor, preferably hemagglutinin, and a differentiation inducer. In the following, it is at times referred to as "the reagent/kit of the present invention for differentiation induction". The E-cadherin inhibitor, hemagglutinin, differentiation inducer and the like used in the reagent/kit of the present invention for differentiation induction are appropriately as described in "1. The differentiation induction method of the present invention".

The E-cadherin inhibitor and the differentiation inducer can be mixed and provided as a single reagent as long as they do not adversely affect each other, or they can be prepared as separate reagents and provided as a kit.

### [Example]

The present invention is described in more detail below with reference to the following examples, but they are merely illustrative and the present invention is not limited to these examples.

### [Example 1] Differentiation induction of iPS cells using hemagglutinin

Differentiation induction of iPS cells using hemagglutinin was performed based on the method described in WO2015/053375, which is specifically as follows.

The reagents used are as follows.
· differentiation induction basic medium (Glasgow's MEM (GMEM) medium (Invitrogen), KnockOut^{™}Serum Replacement (KSR)
(Invitrogen), 0.1 mM MEM non-essential amino acid solution (Invitrogen), 1 mM sodium pyruvate (SIGMA), StemSure
(registered trademark) 10 mmol/l 2-mercaptoethanol (×100) (Wako Pure Chemical Industries, Ltd.), 100 U/ml penicillin-100 µg/ml streptomycin (Invitrogen))
· first differentiation induction medium (differentiation induction basic medium containing 20% KSR, 10 µM Y-27632 (Wako Pure Chemical Industries, Ltd.), 5 µM SB431542 (SIGMA), 3 µM CKI-7 (SIGMA), a given concentration of hemagglutinin (Table 1))
· second differentiation induction medium (differentiation induction basic medium containing 15% KSR, 10µM Y-27632 (Wako Pure Chemical Industries, Ltd.), 5 µM SB431542 (SIGMA), 3 µM CKI-7 (SIGMA), a given concentration of hemagglutinin (Table 1))
· third differentiation induction medium (differentiation induction basic medium containing 10% KSR, 10 µM Y-27632 (Wako Pure Chemical Industries, Ltd.), 5 µM SB431542 (SIGMA), 3 µM CKI-7 (SIGMA), a given concentration of hemagglutinin (Table 1))
· fourth differentiation induction medium (differentiation induction basic medium containing 10% KSR, a given concentration of hemagglutinin (Table 1))
· RPE maintenance medium (67% DMEM low glucose (SIGMA), 29% F12 (SIGMA), 1.9 mM L-glutamine (Invitrogen), 1.9% B-27 supplement (Invitrogen), 96 U/mL penicillin sodium, 96 µg/mL Streptomycin sulfate)

### Production of RPE cells (differentiation induction)

Human skin-derived iPS cells (253G1, provided by Kyoto University) were seeded onto a laminin-coated culture dish (manufactured by Sumika Bakelite Co., Ltd.) at 9×10⁶ cells/9 cm dish. The laminin-coated culture dish was prepared by coating 9 cm culture dish (BD FALCON) with a 0.5 µg/cm² solution of laminin 511E8 fragment (a protein disclosed in Example (3) of WO2011043405, provided by Osaka University) at 37 degrees for 1 hr or longer. The iPS cells quickly adhered to the culture dish, and formation of floating aggregates was not observed.

The first day of culture was set as Day 0, and the entire medium was exchanged every day from the start of culture (Day 1) until around Day 40, when pigment cells were confirmed. The composition of the medium was changed stepwise as shown below: differentiation induction basic medium was used on Day 0, the first differentiation induction medium (20% KSR) on Days 1-4, the second differentiation induction medium (15% KSR) on Days 5-8, the third differentiation induction medium (10% KSR) on Days 9-12, the fourth differentiation induction medium (10% KSR) from Day 13 until around Day 40 when pigment cells were confirmed, and RPE maintenance medium was used until Day 47. The effect of hemagglutinin, added at different concentrations to each medium, on RPE differentiation induction was observed.

The experiment conditions (conditions 1-3) and the results are shown in Table 1. Differentiation induction into RPE cells was successful even without addition of hemagglutinin; however, addition of hemagglutinin moderately loosened the intercellular adhesion of the cell aggregates, the cell aggregates became smaller, and the number of cells that differentiated into RPE cells increased strikingly. On the other hand, the number of fibroblast-like cell groups, which were undesired cells observed together with RPE cells, decreased as hemagglutinin was added.

**[Table 1]**

| experiment conditions | condition 1 | condition 2 | condition 3 |
|---|---|---|---|
| medium | differentiation induction basic medium | | |
| differentiation inducer | with differentiation inducer | with differentiation inducer | with differentiation inducer |
| with or without addition of HA complex | without addition of HA complex | with addition of 10 nM HA complex | with addition of 30 nM HA complex |
| formation of cell aggregates in three-dimensional layer structure | large cell aggregate | small cell aggregate | smaller cell aggregate |
| differentiation into RPE cells (formation of cell aggregates with dark pigments) | observed in some small cell aggregates | many observed in small cell aggregates | more observed in small cell aggregates |
| differentiation into other cells | RPE cell | RPE cell | RPE cell |
| | <<<cell group with fibroblast-like morphology | <<cell group with fibroblast-like morphology | <cell group with fibroblast-like morphology |

### [Industrial Applicability]

The method and composition according to the present invention are useful in, for example, the field of regenerative medicine.

This application is based on a patent application No. 2022-017547 filed in Japan (filing date: February 7, 2022), the contents of which are incorporated in full herein.

## Claims

1. A method for producing a retinal pigment epithelial (RPE) cell, comprising promoting differentiation induction from a pluripotent stem cell into an RPE cell by culturing the pluripotent stem cell in a medium comprising an E-cadherin inhibitor.

2. The method according to claim 1, wherein the pluripotent stem cell is an embryonic stem (ES) cell or an induced pluripotent stem (iPS) cell.

3. The method according to claim 1 or 2, wherein the medium comprises a differentiation inducer.

4. The method according to claim 3, wherein the differentiation inducer is a Nodal signal inhibitor and a Wnt signal inhibitor.

5. The method according to claim 1 or 2, comprising the following steps:
(1) a step of forming an aggregate of pluripotent stem cells, and
(2) a step of culturing the aggregates obtained in step (1) in an RPE cell induction medium comprising an E-cadherin inhibitor.

6. The method according to claim 5, wherein the RPE cell induction medium comprises a Nodal signal inhibitor and a Wnt signal inhibitor.

7. The method according to claim 1, wherein the E-cadherin inhibitor is hemagglutinin.

8. A differentiation induction promoting agent from a pluripotent stem cell into an RPE cell, comprising an E-cadherin inhibitor.

9. The promoting agent according to claim 8, wherein the pluripotent stem cell is an ES cell or an iPS cell.

10. The promoting agent according to claim 8 or 9, wherein the E-cadherin inhibitor is hemagglutinin.

11. A reagent or kit for inducing differentiation of a pluripotent stem cell into an RPE cell, comprising an E-cadherin inhibitor and a differentiation inducer.

12. The reagent or kit according to claim 11, wherein the differentiation inducer is a Nodal signal inhibitor and a Wnt signal inhibitor.

13. The reagent or kit according to claim 11 or 12, wherein the pluripotent stem cell is an ES cell or an iPS cell.

14. The reagent or kit according to claim 11 or 12, wherein the E-cadherin inhibitor is hemagglutinin.
